# EUROPEAN PATENT APPLICATION

(11) **EP 1 281 413 A2**
(43) Date of publication of application: **05.02.2003**
(21) Application number: 02015607.1
(22) Date of filing: 15.07.2002
(51) Int. Cl.: A61M 13/00

(54) **Pneumoperitoneum gas heating and humidifying apparatus and device for inducing pneumoperitoneum**

(30) Priority: 31.07.2001 JP 2001232435; 25.04.2002 JP 2002124188
(71) Applicant: Senko Medical Instrument Mfg. Co., Ltd., Bunkyo-ku Tokyo 169-8925 (JP)
(72) Inventor: Inoue, Masaaki, Senko Medical Instr. Mfg. Co.,Ldt., Tokyo 169-8925 (JP)
(74) Representative: Kuhnen & Wacker

(57) **Abstract**

The present invention aims to provide a pneumoperitoneum gas heating and humidifying apparatus and a pneumoperitoneum device employing this apparatus, in which there is no contamination of the carbon dioxide gas with bacteria, a clean humidified gas is obtained, and the temperature of gas for which the relay was halted and then restarted does not rise excessively. This pneumoperitoneum gas heating and humidifying apparatus provided with an airline, a water conduit which is provided to at least a portion of the outer periphery of this airline, and water supply ports which are connected to the water conduit, wherein at least a portion of the wall of the airline facing the water conduit is formed of a water permeable membrane . The present invention further provides a pneumoperitoneum device in which a carbon dioxide gas cylinder, a gas flow regulator, and a trocar catheter are sequentially connected via piping.

## Description

### BACKGROUND OF THE INVENTION

### Field of the Invention

The present invention relates to a pneumoperitoneum gas heating and humidifying apparatus and to a device for inducing pneumoperitoneum (hereinafter called "pneumoperitoneum device") that are employed in endoscopically-guided surgical procedures within the abdominal cavity.

### Description of the Related Art

Surgery on the abdominal contents conventionally has been performed by making a large incision in the abdomen, a procedure that was both considerably painful and physically stressful for the patient. Post-operative recovery from this procedure was frequently prolonged. Accordingly, the use of endoscopically-guided surgery, in which a small hole is made in the abdominal wall through which an endoscope and surgical instruments are inserted into the abdominal cavity, has spread rapidly in recent years as a minimally invasive alternative to laparotomy.

In order to maintain surgical space and the endoscope's visual field when performing endoscopically-guided abdomenal procedures, it is necessary to expand the abdominal cavity. This is accomplished by supplying carbon dioxide gas into the abdominal cavity and inflating it. The carbon dioxide gas is typically at room temperature, and is a dry gas with a relative humidity near 0%. When carbon dioxide gas is supplied into the abdominal cavity in this form, the patient's body temperature will decrease as a result of contact with the cooler temperature gas. In addition, since the carbon dioxide is a dry gas, it tends to dry the inside of the abdominal cavity, causing moisture to evaporate from the parietal and visceral pleura. This also contributes to further body temperature cooling as heat is released from body surfaces with the evaporation of moisture. Accordingly, the patient not only consumes energy resources due to the surgery, but also must expend energy to maintain body temperature in the face of the above-described temperature-lowering influences. These factors can contribute negatively to the patient's post-operative recovery.

To resolve these problems, attempts have been made to heat and humidify the carbon dioxide gas that is being supplied into the abdominal cavity. Typically, a heating and humidifying apparatus (such as the heated humidifier manufactured by Fisher & Paykel Healthcare Corp. Ltd., for example) is employed in which water within a container is heated using a heating plate, and the carbon dioxide gas is brought into direct contact with this heated water. The Fisher & Paykel Healthcare heated humidifier is intended for use in a ventilator, and is designed to heat and humidify oxygen or air that flows at a constant rate or intermittently at a cycle of 10∼40 times/min. Furthermore, since this heated humidifier is designed for use in a ventilator where it must be capable of heating gas at a relatively high constant flow rate of 5∼50 L/min, it employs a heating plate with a comparatively large thermal capacity, and is designed so that the temperature of the heating plate becomes relatively high.

Given the efficiency of heat transfer between carbon dioxide gas and the water, the water temperature within the heating and humidifying apparatus is set to 40°C or higher in order to raise the temperature of the carbon dioxide gas to be supplied into the abdominal cavity to 37°C when using a heating and humidifying apparatus that employs the above-described heating plate.

However, in a pneumoperitoneum device, the carbon dioxide gas is continuously supplied until the abdominal cavity expands and the internal pressure reaches a preset value, at which point the supply of gas is stopped. Supply of carbon dioxide gas is resumed when the internal pressure falls and the abdominal cavity begins to deflate. Accordingly, the supply of carbon dioxide gas may be halted for a minute or more, depending on the conditions in the abdominal cavity.

Accordingly, when carbon dioxide gas is intermittently supplied into the abdominal cavity using a heating and humidifying apparatus that employs the above-described heating plate, the carbon dioxide gas in the apparatus when gas supply has been stopped continues to be warmed by the water heated by the heating plate, with the temperature of the gas increasing to approximately the same temperature as the water. As a result, even if the power source to the heating plate is cut off when the supply of gas is halted, so that the water inside the container does not lose further heat to the gas, the heating plate's residual heat continues to heat the water. The temperature of the carbon dioxide gas thus becomes higher, sometimes reaching 60°C or more.

Furthermore, in this type of heating and humidifying apparatus, a relatively large gaseous portion of 100 ml or more is present within the container. Moreover, the volume of this gaseous portion gradually increases as the amount of water within the container drops accompanying humidification.

When the pressure inside the abdominal cavity decreases and carbon dioxide gas supply is resumed under these circumstances, a large amount of carbon dioxide gas that has reached high temperatures at the extremes described above is supplied into the abdominal cavity, and a considerable physical stress is placed on the patient during surgery.

In addition, in a heating and humidifying apparatus such as described above that employs a heating plate, the carbon dioxide gas comes in contact with water, and this gas which has been in contact with the water is directly supplied into the abdominal cavity.

The inside of this heating and humidifying apparatus is a high-temperature, highly humid environment, which is very conducive to bacterial growth. Typically, the water employed here is distilled or sterile water so as to limit the effect of administering chlorine or impurities to the patient. If, however, bacteria somehow get introduced into the water, they can grow within the heating and humidifying apparatus. Thus, there is the possibility of infecting the patient since the bacteria could be introduced into the body via the carbon dioxide gas which has been in direct contact with the water.

### SUMMARY OF THE INVENTION

After taking into consideration the above-described circumstances and carrying out extensive research, the present inventors completed this invention with the discovery of a pneumoperitoneum gas heating and humidifying method in which, even in the case where the carbon dioxide gas is intermittently supplied, there is no possibility of supplying a large amount of extremely high temperature gas when reinitiating gas supply, nor of introducing bacteria into the patient.

Namely, the pneumoperitoneum gas heating and humidifying apparatus of the present invention is provided with an airline, a water conduit provided to at least a portion of the outer periphery of this airline, and a water supply port which is connected to the water conduit, wherein at least a portion of the wall of the airline line facing the water conduit is formed of a water vapor permeable membrane.

In the pneumoperitoneum gas heating and humidifying apparatus of this design, the moisture in the water conduit becomes water vapor after passing through a water vapor permeable membrane, and mixes with the carbon dioxide gas in the airline, thereby humidifying the gas. The water in liquid form in the water conduit does not permeate the water vapor permeable membrane, so that there is no direct contact between liquid water and the carbon dioxide gas.

The pneumoperitoneum gas heating and humidifying apparatus of the present invention can employ a design in which the heater for heating the water in the water conduit is provided to an outer pipe that covers the water conduit.

In the pneumoperitoneum gas heating and humidifying apparatus of this design, the water in the water conduit is heated by the heater, and the carbon dioxide gas is then heated by transmission of heat from the heated water through the water vapor permeable membrane

The present invention further provides a pneumoperitoneum device characterized in the sequential connection via piping of a carbon dioxide gas cylinder, a gas flow regulator, a pneumoperitoneum gas heating and humidifying apparatus, and a trocar catheter.

In the pneumoperitoneum device with this design, the amount of carbon dioxide gas supplied from the carbon dioxide gas cylinder can be adjusted, including ceasing supply of the gas, in response to intra-abdominal pressure, and carbon dioxide gas heated and humidified to an appropriate temperature and humidity by the pneumoperitoneum gas heating and humidifying apparatus can be supplied into the abdominal cavity via the trocar catheter.

The trocar catheter is an instrument employed to maintain a port into the body when surgical instruments are going to be frequently passed in and out of the cavity. The trocar catheter is cylindrically-shaped and has a needle with a narrowed tip.

In the pneumoperitoneum device of the present invention, a heat and humidity sensor detector can be provided inside the trocar catheter.

In the pneumoperitoneum device of this design, the temperature and humidity inside the abdominal cavity can be accurately detected.

In this pneumoperitoneum device, a temperature maintaining means for maintaining the temperature of the piping connecting the trocar catheter and the pneumoperitoneum gas heating and humidifying apparatus can be provided.

In the pneumoperitoneum device of this design, the temperature of the carbon dioxide gas which was heated and humidified by the pneumoperitoneum gas heating and humifidifying apparatus does not fall while passing through the piping on route to the trocar catheter. Thus, water vapor does not condense on the inner surface of the piping.

In this case, the temperature maintaining means can be provided to the outside or the inside of the piping that connects the pneumoperitoneum gas heating and humidifying apparatus and the trocar catheter.

### BRIEF DESCRIPTION OF THE DRAWINGS

FIG. 1 is a cross-sectional view of one embodiment of the pneumoperitoneum gas heating and humidifying apparatus of the present invention.
FIG. 2 shows one embodiment of the water vapor permeable membrane tube used for exchanges.
FIG. 3 shows one embodiment of the pneumoperitoneum device of the present invention.
FIG. 4 is a cross-sectional view showing the trocar catheter inserted into the temperature and humidity sensor detector.
FIG. 5 shows the molecular structure of Nafion®.

### PREFERRED EMBODIMENTS OF THE PRESENT INVENTION

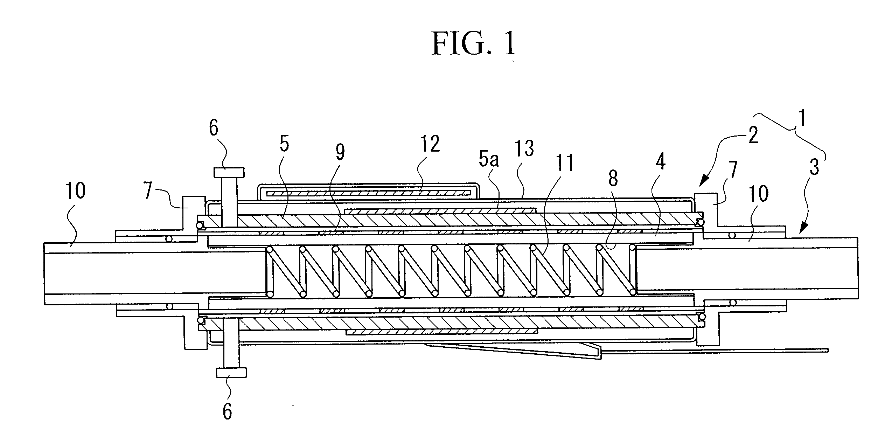

FIG. 1 shows an embodiment of the pneumoperitoneum gas heating and humidifying apparatus according to the present invention. This pneumoperitoneum gas heating and humidifying apparatus 1 is roughly composed of a cylindrically shaped main body 2 and an airline 3 that is inserted into the main body 2.

The main body 2 is provided with an outer casing 5 which surrounds the airline 3 which has been inserted into the main body 2; a water conduit 4 which is formed between the airline 3 and outer casing 5; a planar-shaped heater 5a attached to the surface of the outer casing 5; a pair of water supply ports 6 for permitting water to flow into the water conduit 4; and a pair of caps 7 for supporting both ends of the outer casing 5.

The airline 3 is provided with a protecting pipe 9 consisting of a cylindrically shaped metal mesh disposed to the outermost periphery; a cylindrically shaped water vapor permeable membrane 8 that is inserted into the protecting pipe 9; a pair of supporting members 10 attached to both ends of the water vapor permeable membrane 8 for supporting this water vapor permeable membrane; and a form-retaining spiral 11 that is inserted into the water vapor permeable membrane 8.

The heater 5a is for heating and maintaining the water inside the water conduit 4 to a suitable temperature (set value) by heating the outer casing 5. The water temperature inside the water conduit 4 is measured using a temperature sensor (not shown), and is controlled so as to maintain the set value. Furthermore, the heater 5a is covered with a protective cover 13 so that the person handling pneumoperitoneum gas heating and humidifying apparatus 1 does not accidentally touch the heater 5a.

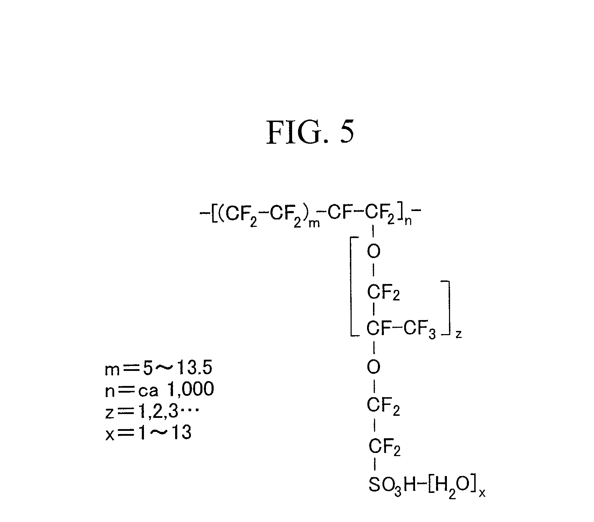

The water vapor permeable membrane 8 is, for example, a thin membrane made of Nafion® (registered trademark of Dupont Corp.) which has the molecular structure shown in FIG. 5. The water vapor permeable membrane 8 has no holes or spaces. Rather, the moisture in the water conduit 4 is transferred in molecular form to the sulfonic acid groups in the membrane, and is released in the form of water vapor into the carbon dioxide gas in the airline 3. Thus, while water vapor is permitted to permeate the membrane, the movement of other gaseous and liquid components is prevented.

Water vapor from the water on the water conduit 4 side passes through the water vapor permeable membrane 8, and is relayed to the carbon dioxide gas in the airline 3, and the carbon dioxide gas is humidified. In this case, it is preferable to make the relative humidity of the carbon dioxide gas, which had been approximately 0% prior to humidification, to be in the range of 90∼100%.

Nafion® was employed for the material of the water vapor permeable membrane 8 in FIG. 1. However, the water vapor permeable membrane 8 is not limited to Nafion®; rather any material is acceptable provided that it has the property of permitting only water vapor to permeate, and of preventing the passage of other substances.

The membrane area of the water vapor permeable membrane 8 is not particularly restricted. Rather, it is acceptable to provide the water vapor permeable membrane 8 having just the area needed to obtain the desired amount of water vapor. For example, when the membrane with an inner diameter of 10 mm is employed for the water vapor permeable membrane 8, the cubic measure of the area through which the gas passes in airline 3 becomes roughly 30 ml to obtain the desired amount of water vapor, which is significantly smaller than a conventional heating and humidifying apparatus employing the heating plate.

The protecting pipe 9 is provided to prevent the person handling the pneumoperitoneum gas heating and humidifying apparatus 1 or a foreign object from coming into contact with the water vapor permeable membrane 8 from the water conduit 4 side and damaging the membrane.

For example, as explained below, the water vapor permeable membrane 8 may be removed from the main body 2 for the each airline 3, and exchanged for a new one or sterilized by autoclaving or plasma irradiation. Even in this case, because the protecting pipe 9 is present, there is no contact between the water vapor permeable membrane 8 and foreign objects or the person handling the penumoperitoneum gas heating and humidifying apparatus 1, so that damage to the water vapor permeable membrane 8 does not occur.

This protecting pipe 9 is formed of a mesh structure, so that the water in the water conduit 4 freely flows inside and outside of the protecting pipe 9. Furthermore, the protecting pipe 9 is formed to prevent a person or foreign object from coming into contact with and damaging the water vapor permeable membrane 8 from the water conduit 4 side, and the flow of water within the water conduit 4 is low, so that even if the mesh openings are relatively small, there is no impediment to water flow. Accordingly, the size and shape of the mesh is not particularly restricted, so that aluminum can be employed for the metal mesh material for forming the protecting pipe 9, for example. It is not absolutely essential that the protecting pipe 9 be made of metal. Rather, it may also be made of a resin mesh that has a degree of flexibility.

The supporting member 10 supports both ends of the protecting pipe 9 and water vapor permeable membrane 8, and forms the wall of the water conduit 4 along with the water vapor permeable membrane 8, cap 7, and outer casing 5, rendering the water conduit 4 watertight. In addition, the supporting member 10 also supports the airline 3 which is inserted inside the main body 2.

The form-retaining spiral 11 which is inserted into the water vapor permeable membrane 8 prevents deformation of the water vapor permeable membrane 8 and maintains its shape. The form-retaining spiral 11 is designed so as not to negatively affect the flow of carbon dioxide gas inside the airline 3. It is not absolutely essential that the form-retaining spiral 11 be in the shape of a spiral; rather, other designs are possible, provided they are effective in preventing deformation of the water vapor permeable membrane 8 and in maintaining its shape.

In this pneumoperitoneum gas heating and humidifying apparatus 1, the water inside the water conduit 4 is heated through the outer casing 5 using the heater 5a. The carbon dioxide gas inside the airline 3 is then heated by transmission of the heat from this heated water through the water vapor permeable membrane 8. In this case, it is preferable to heat the carbon dioxide gas to a temperature of around 37°C. Note that when one takes into consideration the efficiency of thermal transmission from water to carbon dioxide gas, the temperature of the heater 5a must be set to be higher than 37°C. At the same time, it is desirable to provide the heater 5a with an overheating prevention mechanism. For example, it is desirable to enable adjustment of the maximum temperature of heater 5a to be in a suitable temperature range of 40∼60°C by manipulating a controller 12 that is provided on top of the protective cover 13.

The embodiment in FIG. 1 shows an example in which the heater 5a for heating the water in a water conduit is provided inside the main body 2. However, it is also acceptable to heat the water supplied to the water conduit 4 outside the pneumoperitoneum gas heating and humidifying apparatus of the present invention, and then use this water to heat the carbon dioxide gas.

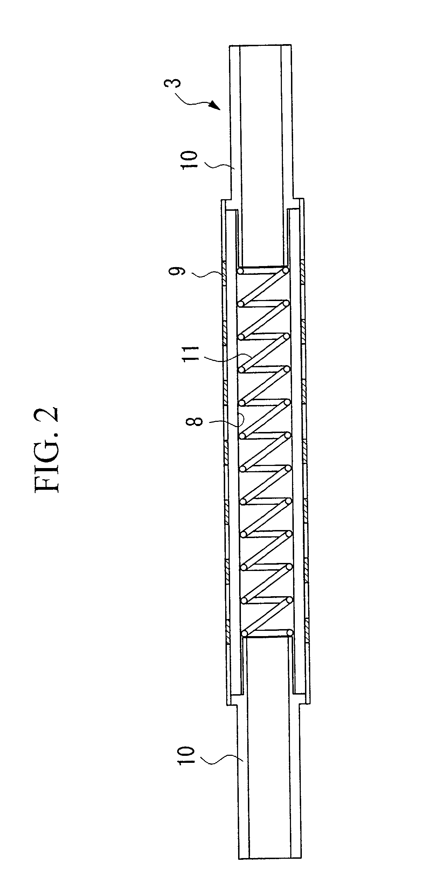

In the pneumoperitoneum gas heating and humidifying apparatus 1 of the present invention, it is desirable that the airline 3 can be releasable and attachable toward the main body 2 as shown in FIG. 2. As a result, the airline 3 can be easily sterilized or exchanged for a new airline. In this case, it is also desirable to always protect water vapor permeable membrane 8 by releasing both itself and the protecting pipe 9 along with the airline 3. In this way, when attaching and releasing, or sterilizing, the airline 3, the operator will not accidentally touch the water vapor permeable membrane 8 and damage it.

Removal of the airline 3 from the main body 2 is performed by pulling off the caps 7 from supporting the members 10 and pulling the airline 3 out from the other portions of the main body 2. Conversely, attachment of the airline 3 is performed by removing the caps 7 from both ends of the main body 2, inserting the airline 3, and then reattaching the caps 7.

Next, the pneumoperitoneum device of the present invention will be explained.

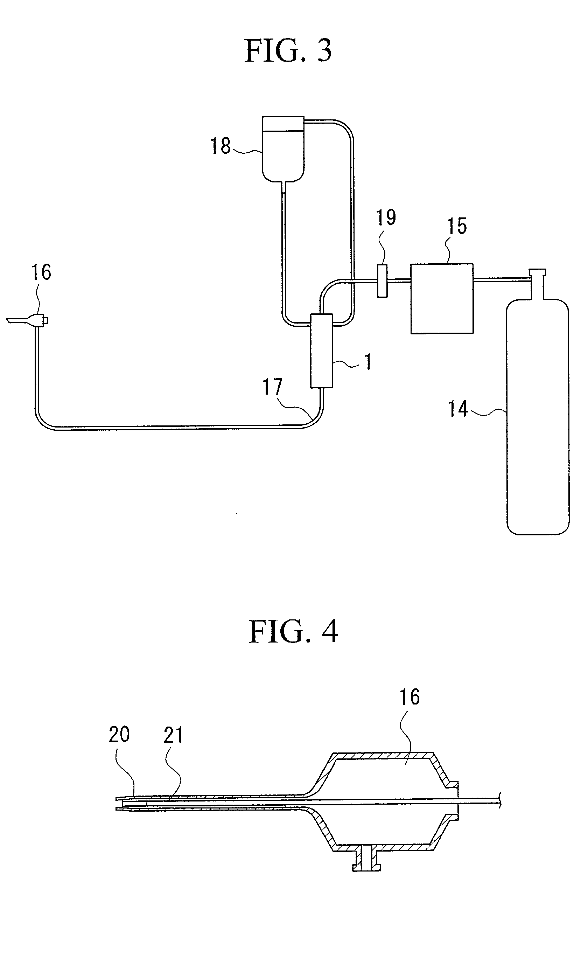

As shown in FIG. 3, this pneumoperitoneum device is formed by sequentially connecting a carbon dioxide gas cylinder 14, a gas flow regulator 15, the above-described pneumoperitoneum gas heating and humidifying apparatus 1, and a trocar catheter 16, by means of piping 17. Pipes consisting of various flexible tubes or hard materials may be suitably employed for the piping 17.

Carbon dioxide gas from the carbon dioxide gas cylinder 14 is supplied into the airline 3 of the pneumoperitoneum gas heating and humidifying apparatus 1 while its flow is adjusted using the gas glow regulator 15 in response to the internal pressure of the peritoneal cavity. The temperature and humidity of the carbon dioxide gas is adjusted using the pneumoperitoneum gas heating and humidifying apparatus 1, and the gas is supplied into the patient's abdominal cavity through the piping 17 and trocar catheter 16.

The distilled water container 18 is set at a position which is 40∼50 cm higher than the pneumoperitoneum gas heating and humidifying apparatus 1. Distilled water is supplied through the piping into the water conduit 4 from one of the water supply ports 6 in the pneumoperitoneum gas heating and humidifying apparatus 1. It is not necessary for the water in the water conduit 4 to be constantly flowing. Rather, only the water lost from the water conduit 4 as a result of the humidification of the carbon dioxide gas need be supplied from the distilled water container 18. Accordingly, as shown in FIG. 3, the other water supply port 6 may be connected to an gaseous portion of the distilled water container 18 through piping, or may simply be sealed.

In the rare event that bacteria or a foreign object has become mixed into the carbon dioxide, it is desirable to provide a bacterial filter 19 for removing bacteria or foreign matter with the piping 17 which connects the gas flow regulator 15 and pneumoperitoneum gas heating and humidifying apparatus 1.

Any type of device may be employed for the gas flow regulator 15, provided it is one that can suitably adjust the carbon dioxide gas flow in response to the value of the pressure within the patient's abdominal cavity. The Laparo CO₂-Pneu 2232 manufactured by the Richard Wolf GmbH may be cited as one example of such a device.

Any trocar catheter employed in endoscopically-guided surgical procedures on abdominal contents may be used for the trocar catheter 16.

As shown in FIG. 4, a temperature and humidity sensor detector may be provided inside trocar catheter 16 as necessary in the pneumoperitoneum device of the present invention. In FIG. 4, a temperature and humidity sensor detector 21 has been inserted into the hollow cavity in the tip 20 of the trocar catheter 16. The end of the detector 21 does not project outside the end of tip 20 of the trocar catheter 16, but rather is contained within the needle. In this way, there is little possibility that the detector will come into contact with abdominal organs and give an incorrect temperature or humidity value.

This temperature and humidity sensor is of a size such that the tip of the sensor which is inserted into the trocar catheter 16 will not impede the flow of carbon dioxide gas inside the trocar catheter 16.

The temperature and humidity detector manufactured by S. K. I. NET, Inc., may be cited as examples of this type of temperature and humidity sensor. The temperature and humidity measured by this temperature and humidity sensor are displayed on a temperature and humidity sensor monitor.

In the pneumoperitoneum device of the present invention, it is desirable to provide a temperature maintaining means for maintaining the temperature of the piping 17 that connects the pneumoperitoneum gas heating and humidifying apparatus 1 and trocar catheter 16. This temperature maintaining means prevents the carbon dioxide gas which has been heated by the pneumoperitoneum gas heating and humidifying apparatus 1 from cooling while traveling through the piping 17, and prevents water vapor from condensing on the inner surface of the piping 17. This temperature maintaining means is preferably a heat insulating material or a temperature maintaining heater.

In this case, for example, the heat insulating material or temperature maintaining heater can be provided to the outer periphery of the piping 17 that connects pneumoperitoneum gas heating and humidifying apparatus 1 and trocar catheter 16. Specifically, the periphery of piping 17 is wrapped with the heat insulating material or heater, or the heat insulating material or heater is imbedded into piping 17.

Alternatively, a heater may also be inserted inside the piping 17 that connects the pneumoperitoneum gas heating and humidifying apparatus 1 and trocar catheter 16.

A heating wire or the like may be employed for the above-described heater, for example.

Note that the present invention is not limited to the embodiments described above. Rather, provided they do not depart from the spirit of the invention, various modifications may be optionally employed to meet the requirements of the intended use.

The pneumoperitoneum gas heating and humidifying apparatus of the present invention selectively permits the passage of water vapor and prevents the passage of other components. As a result, there is no possibility of introduction of bacteria as see in conventional heating and humidifying apparatuses in which there is direct contact between the carbon dioxide gas and the water. Thus, clean, humidified gas is obtained without the fear of infection of the patient. The rise in humidity is also extremely fast in the present invention's apparatus. In addition, the volume of the area through which the gas passes in the pneumoperitoneum gas heating and humidifying apparatus is very small. As a result, after the supply of carbon dioxide gas has been stopped due to an increase in intra-abdominal pressure and then reinitiated when the pressure begins to fall, the temperature of this carbon dioxide gas which is to be newly supplied does not increase excessively. Accordingly, unnecessary physical stress to the patient is avoided.

The pneumoperitoneum device of the present invention can supply heated, humidified, clean carbon dioxide gas to the patient's abdominal cavity, so that there is no drying or excessive temperature variations in the abdominal cavity. As a result, unnecessary physical stress to the patient is avoided. Further, since the volume of the area through which the gas passes is extremely small, temperature variations in the carbon dioxide gas which accompany changes in the amount of carbon dioxide gas supplied, chief among these being the cessation of carbon dioxide gas supply to the abdominal cavity, are limited and the stress to the patient is reduced.

When a temperature and humidity sensor is inserted into a trocar catheter in the pneumoperitoneum device of the present invention, the temperature and humidity inside the abdominal cavity can be accurately measured during the endoscopically-guided procedure on the abdominal contents.

In addition, by providing a temperature maintaining means for maintaining the temperature of the piping that connects the pneumoperitoneum gas heating and humidifying apparatus and the trocar catheter, the temperature of the carbon dioxide gas that was heated and humidified in the heating and humidifying apparatus does not decrease as it passes through the piping on its way to the trocar catheter. Thus, water vapor does not condense on the inner surface of the piping.

## Claims

1. A pneumoperitoneum gas heating and humidifying apparatus comprising: an airline, a water conduit that is provided to at least a portion of the outer periphery of said airline, and a water supply port that is connected to said water conduit; wherein at least a portion of the wall of said airline facing said water conduit is formed of a water vapor permeable membrane.

2. A pneumoperitoneum gas heating and humidifying apparatus according to claim 1, wherein a heater for heating the water in said water conduit is provided to an outer piping that covers said water conduit.

3. A pneumoperitoneum device wherein a carbon dioxide gas cylinder, a gas flow regulator, said pneumoperitoneum gas heating and humidifying apparatus according to claim 1, and a trocar catheter are connected sequentially by means of piping.

4. A pneumoperitoneum device wherein a carbon dioxide gas cylinder, a gas flow regulator, said pneumoperitoneum gas heating and humidifying apparatus according to claim 2, and a trocar catheter are connected sequentially by means of piping.

5. A pneumoperitoneum device according to claim 3, wherein a temperature and humidity detector is provided inside said trocar catheter.

6. A pneumoperitoneum device according to claim 4, wherein a temperature and humidity detector is provided inside said trocar catheter.

7. A pneumoperitoneum device according to claim 3, wherein a temperature maintaining means for maintaining the temperature of the piping that connects said pneumoperitoneum gas heating and humidifying apparatus and said trocar catheter is provided.

8. A pneumoperitoneum device according to claim 4, wherein a temperature maintaining means for maintaining the temperature of the piping that connects said pneumoperitoneum gas heating and humidifying apparatus and said trocar catheter is provided.

9. A pneumoperitoneum device according to claim 5, wherein a temperature maintaining means for maintaining the temperature of the piping that connects said pneumoperitoneum gas heating and humidifying apparatus and said trocar catheter is provided.

10. A pneumoperitoneum device according to claim 6, wherein a temperature maintaining means for maintaining the temperature of the piping that connects said pneumoperitoneum gas heating and humidifying apparatus and said trocar catheter is provided.

11. A pneumoperitoneum device according to claim 7, wherein said temperature maintaining means is provided to the outer periphery of the piping that connects said pneumoperitoneum gas heating and humidifying apparatus and said trocar catheter.

12. A pneumoperitoneum device according to claim 8, wherein said temperature maintaining means is provided to the outer periphery of the piping that connects said pneumoperitoneum gas heating and humidifying apparatus and said trocar catheter.

13. A pneumoperitoneum device according to claim 9, wherein said temperature maintaining means is provided to the outer periphery of the piping that connects said pneumoperitoneum gas heating and humidifying apparatus and said trocar catheter.

14. A pneumoperitoneum device according to claim 10, wherein said temperature maintaining means is provided to the outer periphery of the piping that connects said pneumoperitoneum gas heating and humidifying apparatus and said trocar catheter.

15. A pneumoperitoneum device according to claim 7, wherein said temperature maintaining means is provided to the inside of the piping that connects said pneumoperitoneum gas heating and humidifying apparatus and said trocar catheter.

16. A pneumoperitoneum device according to claim 8, wherein said temperature maintaining means is provided to the inside of the piping that connects said pneumoperitoneum gas heating and humidifying apparatus and said trocar catheter.

17. A pneumoperitoneum device according to claim 9, wherein said temperature maintaining means is provided to the inside of the piping that connects said pneumoperitoneum gas heating and humidifying apparatus and said trocar catheter.

18. A pneumoperitoneum device according to claim 10, wherein said temperature maintaining means is provided to the inside of the piping that connects said pneumoperitoneum gas heating and humidifying apparatus and said trocar catheter.
